# EUROPEAN PATENT SPECIFICATION

(11) **EP 4 326 698 B1**
(45) Date of publication and mention of the grant of the patent: **04.06.2025**
(21) Application number: 22724007.4
(22) Date of filing: 20.04.2022
(51) Int. Cl.: C07C 29/132, C07C 29/60, C07C 31/20, C07C 31/22, C01B 3/48, C01B 3/56

(54) **PROCESS AND EQUIPMENT FOR PRODUCING GLYCOLS FROM CARBOHYDRATES AND HYDROGEN WITH REACTOR OFF-GAS TREATMENT AND HYDROGEN PRODUCTION**
VERFAHREN UND AUSRÜSTUNG ZUR HERSTELLUNG VON GLYKOLEN AUS KOHLENHYDRATEN UND WASSERSTOFF MIT REAKTORABGASBEHANDLUNG UND WASSERSTOFFHERSTELLUNG
PROCÉDÉ ET ÉQUIPEMENT DE PRODUCTION DE GLYCOLS À PARTIR À PARTIR DE GLUCIDES ET D'HYDROGÈNE AVEC TRAITEMENT DES GAZ DE RÉACTEUR ET PRODUCTION D'HYDROGÈNE

(30) Priority: 21.04.2021 EP 21169656
(43) Date of publication of application: 28.02.2024
(73) Proprietor: Avantium Knowledge Centre B.V., 1014 BV Amsterdam (NL)
(72) Inventor: ANSOVINI, Davide, 1014 BV Amsterdam (NL); DAMEN, Kay Jochem, 1014 BV Amsterdam (NL)
(74) Representative: Avantium Intellectual Property
(86) International application number: PCT/EP2022/060352
(87) International publication number: WO 2022/223577

(56) References cited:
- WO-A1-2014/048860
- WO-A1-2016/114661
- US-A1- 2008 025 903
- US-A1- 2008 274 019
- US-A1- 2016 244 555
- HE FENG ET AL: "Hydrogen production from methane and solar energy - Process evaluations and comparison stu", INTERNATIONAL JOURNAL OF HYDROGEN ENERGY, ELSEVIER SCIENCE PUBLISHERS B.V., BARKING, GB, vol. 39, no. 31, 14 June 2014 (2014-06-14), pages 18092 - 18102, XP029048022, ISSN: 0360-3199, DOI: 10.1016/J.IJHYDENE.2014.05.089

## Description

### Introduction

The present invention relates to a process and equipment for producing glycols from a carbohydrate source and hydrogen, which process can be carried out continuously in a reactor. The equipment and process further relate to hydrogen production to produce hydrogen for use in the reaction and to treatment of the off-gas from the reactor.

### Background of the invention

WO 2016/114661 discloses a continuous process for preparation of ethylene glycol from a carbohydrate source. Said process is carried out in a stirred tank reactor (CSTR) in which a catalyst system is present and to which hydrogen is being fed. Said catalyst system comprises a tungsten compound and at least one hydrogenolysis metal.

Hydrogen required for the above process can be provided in any conventional way. For example, steam methane reforming (or short: steam reforming) is a well known chemical reaction for producing syngas (a mixture of mainly hydrogen and carbon monoxide). It requires methane low in sulfur, usually desulfurised natural gas, to prevent catalyst poisoning. It is also a strongly endothermic reaction, requiring lots of heat. Hence, a combustible gas (typically natural gas) is usually burned with air for such. Frequently, such steam reforming is combined with a water-gas shift reaction to further increase hydrogen yield, and producing next to hydrogen carbon dioxide. The reaction is mildly exothermic. The hydrogen from such mixture of mainly hydrogen and carbon dioxide can be separated from the carbon dioxide by e.g. pressure swing adsorption, yielding, next to hydrogen in the required purity (typically > 99%, e.g. 99.9% or even 99.99%) a tail gas comprising carbon dioxide and some minor gasses. A combination of steam methane reforming (SMR) with water gas shift reaction (WGS) and pressure swing adsorption (PSA) is well known. However, operating such processes is expensive, especially on small and medium scale, and it has a substantial influence on how attractive processes like that in WO 2016/114661 are from a commercial point of view. Hence, there is a desire for a hydrogen production that can provide hydrogen to the process of WO 2016/114661 and the like in an economic way.

In the processes of WO 2016/114661 and the like the reactor produces a liquid effluent comprising e.g. the desired ethylene glycol, other glycols such as propylene glycol, butanediol, polyols, simple alcohols and water. Clearly, for a commercial operation such process will comprises downstream processing involving purification of the liquid product flow out of the reactor, so that the desired ethylene glycol and/or propylene glycol and possibly polyols can be obtained in sufficient purity. Usually, such process involves distillation, usually multiple distillation steps. Such distillation is described e.g. in EP3280692 and EP3126315. Such distillation requires heating of the streams fed to the distillation columns. Typically, ethylene glycol is the most desired product, but also side products like propylene glycol and/or glycerol, provided they can be isolated in desired purities, can also be commercially attractive products. CN 102643165A discloses a process for producing ethylene glycol (and 1,2-propylene glycol) from sugars by a continuous process through hydrocracking of the sugars using hydrogen and a catalyst system. The reactor effluent is separated into product streams using rectification to obtain the desired products, and at least part of the heavy polyols that are produced are in this process are recycled back to the reactor, together with unreacted sugars. US2016/0244555 discloses a process for producing glycols from a liquid sugar stream derived from a ligno-cellulosic biomass feedstock. Preferably, all the hydrogen used in the conversion process is generated by aqueous phase reforming of the high boiling polyols mixture.

The processes to produce ethylene glycol from a carbohydrate source with hydrogen also produces an off-gas from the reactor, which comprises e.g. excess hydrogen, alkanes such as methane and ethane, and potentially other gasses. Clearly, this has to be dealt with. One option is burning.

A lot of research so far for the type of reactions concerned have focusses on e.g. type of catalysts, type of reactors, and the distillation steps required. However, for these processes to be commercially viable, the various needs of the process (e.g. energy and reactants) and waste streams need to be considered, which so far have received less attention, preferably taking into account capital expenditure and/or operating cost.

Hence, there is a need for a process for producing ethylene glycol and/or propylene glycol and/or glycerol from carbohydrates and hydrogen with a catalyst, preferably in a continuous way, in which process attention is given to providing hydrogen in a way which is believed to make it commercially attractive. Furthermore a process with flexibility is desired as to operating conditions and feedstock.

### Summary of the invention

It has now been found that such can be achieved, at least in part by a process for producing ethylene glycol and/or propylene glycol and/or glycerol in a continuous manner from a feed to a reactor, the feed comprising a carbohydrate source in an aqueous liquid, the reactor further being fed with hydrogen gas, the reactor comprising a catalyst system, said catalyst system comprising a tungsten-containing compound and a hydrogenolysis metal selected from the groups 8, 9 or 10 of the Periodic Table of Elements, wherein a liquid product flow out of the reactor comprises glycols and one or more polyols, and the reactor further provides an off-gas comprising hydrogen and methane,
wherein the hydrogen gas being fed to the reactor is provided by a hydrogen production section comprising:
   - a steam methane reformer (SMR) unit,
   - a water gas shift reactor (WGS) unit,
   - a pressure swing adsorption (PSA) unit,
wherein the feed to the PSA unit comprises the hydrogen-comprising product stream of the WGS unit and the off-gas of the reactor, and
wherein the PSA unit produces a hydrogen product stream comprising at least 95% by volume of hydrogen for feeding to the reactor and a PSA tail gas, wherein the PSA tail gas comprises between 5 and 60% by weight of C1-C3 alkanes which PSA tail gas is fed to the SMR to fuel the steam reforming process in the SMR unit.

The invention further relates to the arrangement of processing equipment for executing the process of this invention. Hence, the invention further relates to processing equipment for producing ethylene glycol and/or propylene glycol and/or glycerol in a continuous manner from a feed comprising a carbohydrate source in an aqueous liquid and hydrogen, the processing equipment comprising:
- a reactor producing a liquid product flow out of the reactor and an off-gas,
- a downstream purification section comprising one or more distillation and/or evaporation units for subjecting the liquid product flow out of the reactor to one or more distillation and/or evaporation steps,
- a hydrogen production section comprising:
   o a steam methane reformer (SMR) unit,
   o a water gas shift reactor (WGS) unit,
   o a pressure swing adsorption (PSA) unit,

wherein the feed to the PSA unit comprises the hydrogen-comprising product stream of the WGS unit and the off-gas of the reactor, and
wherein the PSA unit produces a hydrogen product stream comprising at least 98% by volume of hydrogen for feeding to the reactor and a PSA tail gas, wherein the PSA tail gas comprises between 5 and 60% by weight of C1-C3 alkanes which PSA tail gas is fed to the SMR to fuel the steam reforming process in the SMR unit.

### Detailed description of the invention

As mentioned, the combination of a steam methane reforming and high temperature water gas shift reaction to prepare a gas mixture containing hydrogen, which is subjected to a separation process like pressure swing adsorption to provide hydrogen in sufficient purity is known as such. However, inventors have found that such process becomes economically more attractive in the process of (catalytically) converting carbohydrates with hydrogen into glycols when the off-gas of the reactor is integrated in the design of the process units and how they are linked together. This is due to e.g. the specific composition of the off-gas which contains substantial amounts of hydrogen which go to waste if burned, but also substantial amounts of combustible side products of the reaction. Also, the fact that the precise composition of the off-gas may vary over time or depend on reaction conditions or feedstock fits with the now claimed process. The process arrangement of the present invention may provide a further benefit in that it enhances process flexibility, in that the operators do not have to worry much about the off-gas and how much valuable hydrogen exits the reactor as part of the off-gas.

In the process and equipment according to the invention, the gas produced by the PSA unit and fed-back to the reactor preferably contains a high amount of hydrogen. However, small amounts of e.g. lower alkanes out of the off-gas which are part of the hydrogen stream from PSA back to the reactor may be tolerated. Such other components present may be due to e.g. incomplete separation by the PSA unit. Nevertheless, e.g. for process efficiency it is preferred if the PSA separation is such that the PSA unit produces a hydrogen product stream comprising at least 97% by volume of hydrogen for feeding to the reactor, preferably at least 98% by volume of hydrogen for feeding to the reactor, more preferably at least 99% by volume.

In the process of the invention it is quite easy to reduce emissions of carbon dioxide, which inevitably occur with the water gas shift reaction for hydrogen production. In the presently claimed process, it is preferred to have a form of carbon dioxide capture present after the water gas shift reactor unit. Typically, such carbon dioxide capture unit will be prior to the pressure swing adsorption. Various forms of carbon dioxide capture exist, but it was found that one form is specifically suited in the current invention, which is the technique of carbon dioxide capture with the absorbent methyldiethanol amine (MDEA), as this requires low pressure steam (e.g. 2-7 bara) for the regeneration column, and such low pressure steam may be a waste stream from higher pressure steam being used for providing heat to e.g. reactor or reboiler, or it may be taken from a boiler that is used to burn waste polyols, or it may be taken from the SMR or WSG units. Hence, it is preferred in the present invention that the process further comprises carbon dioxide capture to capture at least part of the carbon dioxide produced by the hydrogen production section, the carbon dioxide capture comprising absorption of carbon dioxide with methyldiethanol amine and regeneration of said methyldiethanol amine using steam.

Likewise, it is preferred for the equipment according to the present invention that the processing equipment according to the invention further comprises a carbon dioxide capture unit comprising an absorption column and a regeneration column and which unit comprises methyldiethanol amine. Preferably, the methyldiethanol amine is regenerated using steam at a pressure of between 2 and 7 bara, even more preferably between 3 and 6 bara.

A further benefit of the process of the present invention is that on the one hand presence of steam at a pressure of e.g. between 5 and 70 bara, preferably at a pressure of between 10 and 50 bara is desired and on the other hand the steam methane reformer and the water gas shift reactor may produce steam. Such steam is desired e.g. to heat the reactor and/or for use downstream of the reactor, e.g. in separation processes, such as evaporators and/or reboilers of distillation columns. The above means that less auxiliary steam is needed to drive the reactor and/or downstream separation or purification processes when the hydrogen production is integrated into the carbohydrate conversion in the present invention. In other words: there is a link between hydrogen production and carbohydrate conversion that suits both these process parts, making it easier to make the overall process economically viable. Thus, in the process of the present invention it is preferred that the steam methane reformer (SMR) produces steam at a pressure of between 5 and 70 bara, preferably between 10 and 50 bara. Likewise, it is preferred that for the processing equipment according to the present invention that the SMR produces, next to syngas, steam at a pressure of between 5 and 70 bara, preferably between 10 and 50 bara. As to the amount of steam desired, such depends on the usage of the steam in other parts of the process, e.g. hydrogenolysis reactor, evaporation, and/or distillation. The amount of steam produced is linked to the amount of hydrogen produced by the combination of steam methane reformer and the water-gas shift reactor, and typically may be, when expressed relative to the amount of monoethylene glycol produced by the process of the invention, between 0.5 and 2 ton steam per ton mono-ethylene glycol produced, and typically between 1 and 1.9 ton steam per ton mono-ethylene glycol produced. These amounts of steam preferably relate to steam at a pressure of between 5 and 70 bara, more preferably to steam at a pressure of between 10 and 50 bara. The temperature of such steam is preferably between 150°C and 290°C, more preferably between 180°C and 270°C (typically for saturated steam, such values may be 20°C higher for superheated steam).

The process for converting a carbohydrate source with hydrogen with a catalytic system to glycols, e.g. ethylene glycol, usually will result in a product mixture comprising monoethylene glycol, propylene glycol, other glycols such as butanediol and pentanediol, and polyols like glycerol, sorbitol and erythritol, but also lower alkanols like methanol and/or ethanol. Depending upon e.g. amounts produced and economic value one or more of these components will have to be isolated from the liquid product stream from the reactor, and minor products will have to be removed. Typically, such will need to be done quite rigorously, as one area of application of e.g. monoethylene glycol is in polyesters such as PET, and for such polyesters high purities are desired for reasons of product properties. Following this, the whole operation of producing glycols or glycerol according to the present invention will require more or less extensive downstream processing (downstream vis a vis the reactor) for removing e.g. water and/or volatiles and/other undesired glycols like butanediol, pentanediol, high-boiling glycols like erythritol and sorbitol. Hence, it is preferred in the process according to the present invention that the liquid product flow out of the reactor is subjected to one or more purification steps comprising one or more distillation steps and/or evaporation steps.

Hence, the processing equipment according to the present invention preferably contains equipment for removing such components, like evaporators and/or distillation columns, e.g. provided with reboilers. Typically, such purification techniques require heat, which can be provided in the form of steam. Conveniently, the interlink between the manufacturing (of glycols from carbohydrates) including purification can utilize steam, whereas the hydrogen production side of the operation produces such steam. Hence, in the present invention it is desired that in the processing equipment according to the present invention the steam produced (e.g. in the hydrogen production) is used for heating the reactor and/or for providing heat to one or more units of the downstream purification section.

As mentioned, the downstream processing may involve multiple distillation columns, for e.g. removing the various undesired glycols and separate the desired ones. This usually results in a bottoms stream at the end of such distillation sequence which contains e.g. a substantial amount of high boiling polyols, such as erythritol and sorbitol. A convenient way of dealing with such is subject such bottoms stream to burning. Since such may be done in a boiler capable of producing steam, and as steam is desired in other parts of the process, also burning of one or more bottom stream (of distillation) is preferably done in a boiler capable of producing steam at a desired pressure. Hence, in the present invention it is preferred that the processing equipment according to the present invention further comprises a boiler for burning the bottom streams of one or more distillation steps, which boiler produces steam in doing so, at a pressure of between 5 and 70 bara, preferably between 10 and 50 bara. And likewise, in the process according to the present invention it is preferred that one or more bottoms streams of distillation comprise polyols, and wherein one or more of these bottom streams is subjected to burning in a boiler to produce steam at a pressure of between 5 and 70 bara, preferably between 10 and 50 bara. Similarly, in the process of the present invention it is preferred that the steam produced by the SMR is used for heating the reactor and/or for providing heat to one or more units of the purification steps.

In order for the hydrogenolysis reactor to work efficiently, and for the hydrogen production according to the present invention to work efficiently, it is preferred that the amount of off-gas from the reactor has a specific weight in ton / ton of monoethylene glycol produced in the process of between 0.05 and 0.70 ton / ton MEG, preferably between 0.07 and 0.40 in ton / ton of monoethylene glycol produced

The process according to the present invention is preferably carried out in a continuous way. A suitable reactor system for the now claimed process comprises a continuously stirred tank reactor (CSTR). Preferably the process is conducted in a reactor system which is a CSTR.

The process of the now claimed invention can suitably be carried out using a catalyst system comprising two co-catalysts. One of the components is preferably a homogeneous catalyst component comprising a tungsten-containing component. A preferred tungsten-containing compound in the catalyst system in the process of the present invention comprises tungstic acid (H₂WO₄), a tungstate salt, tungsten oxide or mixtures thereof, wherein the tungstate salt is preferably an alkali metal tungstate. More preferably, the tungsten-containing compound in the reactor system comprises tungstic acid (H₂WO₄), a tungstate salt, tungsten oxide or mixtures thereof, wherein the tungstate salt is preferably an alkali metal tungstate.

Such homogeneous catalyst in the presently claimed process usually leaves the reactor as part of the liquid product flow. For maintaining a more or less constant concentration of such catalyst it is required that such homogeneous catalyst compound is continuously or periodically added to the reactor. Hence, in the process of the invention it is preferred that the said tungsten-containing compound of the catalyst system is continuously or periodically added to the reactor.

In the process according to the present invention it is preferred that the hydrogenolysis metal selected from the groups 8, 9 or 10 of the Periodic Table of Elements is selected from the group consisting of Cu, Fe, Ni, Co, Pd, Pt, Ru, Rh, Ir, Os, and combinations thereof, and which is preferably selected from Ru and Rh.

### Example

### Example 1 - simulation without carbondioxide capture

A process simulation was run using Aspen Plus (version 11) on a process covering production of ethylene glycol from a feedstock of sucrose (in water) using a catalyst system of a heterogeneous catalyst of ruthenium on a carrier and a homogeneous catalyst comprising tungstic acid.

Hydrogen required for the reaction was simulated to be produced by a combination of steam methane reformer and a high temperature water-gas shift reactor, and retrieved from the off-gas from the reactor by separation in a pressure swing adsorption (PSA). The tail-gas from the PSA unit in the simulation was fed to the steam methane reformer as fuel. Desulfurized natural gas served as source for the hydrogen in the steam methane reformer in the simulation.

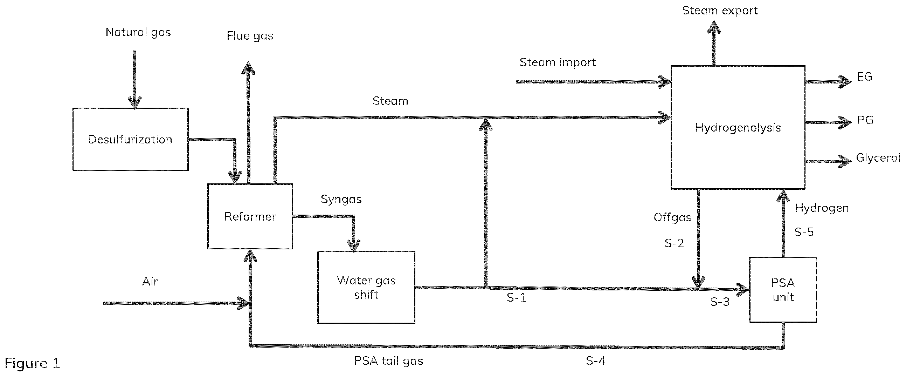

The amount of steam produced at a medium pressure by the steam methane reformer and by the high temperature water gas shift reactor was modelled. Such steam can be used for various purposes in the reaction process (e.g. heating the reactor or medium) and downstream (e.g. evaporation or distillation). The set-up of the various elements is schematically given in figure 1.

For the simulation, among the assumptions that were made, the hydrogen recovery efficiency of the PSA: 86.5%.

Steam production was aimed in the simulation at 26 bara and a temperature of 256°C.

The weight of the streams S-1 to S-5, relative to the amount of MEG produced, is given in table 1.

**Table 1: weight of streams S-1 to S-5, on MEG produced**

| stream | description | Specific mass (ton/ton MEG) |
|---|---|---|
| S-1 | From water-gas shift / water removal | 0.914 |
| S-2 | Off-gas from hydrogenolysis reactor | 0.134 |
| S-3 | Combined S-1 and S-2 | 1.048 |
| S-4 | PSA tail gas | 0.833 |
| S-5 | Hydrogen to hydrogenolysis reactor | 0.164 |

The composition of each of the streams S-1, S-2, S-3, S-4, and S-5 is, in relative terms, given in table 2.

**Table 2: mass fractions streams S-1 to S-5**

| component | S-1 mass fraction | S-2 mass fraction | S-3 mass fraction | S-4 mass fraction | S-5 mass fraction |
|---|---|---|---|---|---|
| C2-C6 diols | | 1.12E-5 | 1.43E-6 | 1.70E-6 | |
| Aliphatic alcohols | | 0.0098 | 0.0013 | 0.0015 | |
| Methane | 0.0484 | 0.5001 | 0.1064 | 0.1259 | 0.0029 |
| Ethane | | 0.0519 | 0.0067 | 0.0079 | |
| Propane | | 0.0179 | 0.0024 | 0.0027 | |
| Butane | | 0.0054 | 0.0007 | 0.0008 | |
| H₂ | 0.1482 | 0.4071 | 0.1814 | 0.0291 | 0.9980 |
| N₂ | 0.0074 | 0.0002 | 0.0066 | 0.0078 | 0.0001 |
| Water | 0.0036 | 0.0075 | 0.0041 | 0.0049 | |
| CO | 0.0925 | | 0.0806 | 0.0957 | |
| CO₂ | 0.6996 | | 0.6099 | 0.7238 | |

The resulting steam production (at 26 bara) was 1.874 ton / ton monoethylene glycol produced.

The main heat input/output of the simulation was as in table 3, based on assumptions:
Flue gas exit temperature: 150°C.
Boiler feed water: 105°C.

**Table 3: heat input/output overview**

| section | Specific heat required (MJ/ton EG) | Specific heat released (MJ/ton EG) |
|---|---|---|
| Natural gas (desulphurised) and reforming section | 6366 | |
| PSA tail gas combustion | | 9417 |
| Water gas shift | | 2052 |

The heat recovery through steam generation: 4660 MJ/ton.

### Example 2 - simulation with carbon dioxide capture

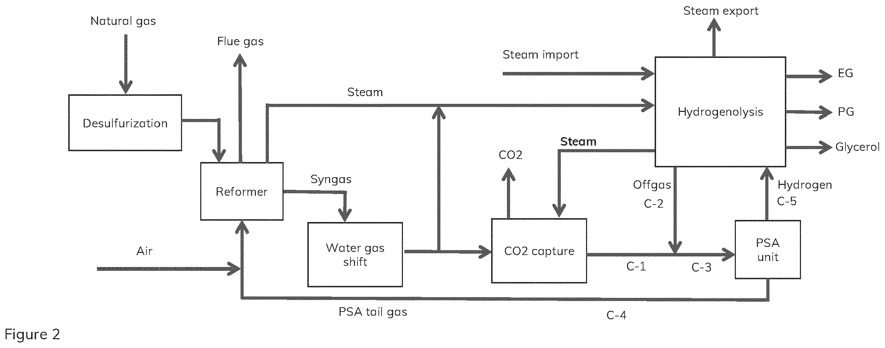

The simulation of example 1 was repeated, expect that the gas composition from the water gas shift reactor (S-1 in example 1) is subjected to a carbon dioxide capture (resulting in stream C-1), so as to reduce the emission of carbon dioxide. The set-up of the various elements is schematically given in figure 2.

For the simulation, the following assumption was made:
Hydrogen recovery efficiency of the PSA: 86.5%.
CO₂ capture efficiency is assumed to be 90%
Steam required for the carbon dioxide captures was low pressure (about 4 bara) available after using high pressure steam in other parts of the process.

Steam production was aimed in the simulation at 26 bara and a temperature of 256°C.

The weight of the streams C-1 to C-5, relative to the amount of MEG produced, is given in table 3.

**Table 3: weight of streams C-1 to C-5, on MEG produced**

| stream | Description | Specific mass (ton/ton MEG) |
|---|---|---|
| C-1 | From CO₂ capture unit | 0.338 |
| C-2 | Off-gas from hydrogenolysis reactor | 0.134 |
| C-3 | Combined S-1 and S-2 | 0.473 |
| C-4 | PSA tail gas | 0.308 |
| C-5 | Hydrogen to hydrogenolysis reactor | 0.164 |

The composition of each of the streams C-1, C-2, C-3, C-4, and C-5 is, in relative terms, given in table 4.

**Table 4: mass fractions streams C-1 to C-5**

| component | C-1 mass fraction | C-2 mass fraction | C-3 mass fraction | C-4 mass fraction | C-5 mass fraction |
|---|---|---|---|---|---|
| C2-C6 diols | | 1.12E-5 | 3,17E-6 | 4.87E-6 | |
| Aliphatic alcohols | | 0.0098 | 0.0028 | 0.0043 | |
| Methane | 0.1308 | 0.5001 | 0.2358 | 0.3610 | 0.0020 |
| Ethane | | 0.0519 | 0.0148 | 0.0227 | |
| Propane | | 0.0179 | 0.0051 | 0.0078 | |
| Butane | | 0.0054 | 0.0015 | 0.0024 | |
| H₂ | 0.4003 | 0.4071 | 0.4022 | 0.0834 | 0.9980 |
| N₂ | 0.0203 | 0.0002 | 0.0145 | 0.0223 | 0.0001 |
| Water | 0.0098 | 0.0075 | 0.0092 | 0.0141 | |
| CO | 0.2499 | | 0.1789 | 0.2746 | |
| CO₂ | 0.1889 | | 0.1352 | 0.2076 | |

The resulting steam production (at 26 bara) was 1.874 ton / ton monoethylene glycol produced.

## Claims

1. Processing equipment for producing ethylene glycol and/or propylene glycol and/or glycerol in a continuous manner from a feed comprising a carbohydrate source in an aqueous liquid and hydrogen, the processing equipment comprising:
- a reactor producing a liquid product flow out of the reactor and an off-gas,
- a downstream purification section comprising one or more distillation and/or evaporation units for subjecting the liquid product flow out of the reactor to one or more distillation and/or evaporation steps,
- a hydrogen production section comprising:
o a steam methane reformer (SMR) unit,
o a water gas shift reactor (WGS) unit,
o a pressure swing adsorption (PSA) unit,
wherein the feed to the PSA unit comprises the hydrogen-comprising product stream of the WGS unit and the off-gas of the reactor, and
wherein the PSA unit produces a hydrogen product stream comprising at least 95% by weight of hydrogen for feeding to the reactor and a PSA tail gas, wherein the PSA tail gas comprises between 5 and 60% by weight of C1-C3 alkanes which PSA tail gas is fed to the SMR to fuel the steam reforming process in the SMR unit.

2. Processing equipment according to claim 1, wherein the equipment further comprises a carbon dioxide capture unit comprising an absorption column and a regeneration column and which unit comprises methyldiethanol amine.

3. Processing equipment according to claim 1 or claim 2, wherein the SMR produces, next to syngas, steam at a pressure of between 5 and 70 bara, preferably between 10 and 50 bara.

4. Processing equipment according to any of the preceding claims, wherein the equipment further comprises a boiler for burning the bottom streams of one or more distillation steps, which boiler produces steam in doing so, at a pressure of between 5 and 70 bara, preferably between 10 and 50 bara.

5. Processing equipment according to any claims 3 or 4, wherein the steam produced is used for heating the reactor and/or for providing heat to one or more units of the downstream purification section.

6. A process for producing ethylene glycol and/or propylene glycol and/or glycerol in a continuous manner from a feed to a reactor, the feed comprising a carbohydrate source in an aqueous liquid, the reactor further being fed with hydrogen gas, the reactor comprising a catalyst system, said catalyst system comprising a tungsten-containing compound and a hydrogenolysis metal selected from the groups 8, 9 or 10 of the Periodic Table of Elements, wherein a liquid product flow out of the reactor comprises glycols and one or more polyols, and the reactor further provides an off-gas comprising hydrogen and methane,
wherein the hydrogen gas being fed to the reactor is provided by a hydrogen production section comprising:
o a steam methane reformer (SMR) unit,
o a water gas shift reactor (WGS) unit,
o a pressure swing adsorption (PSA) unit,
wherein the feed to the PSA unit comprises the hydrogen-comprising product stream of the WGS unit and the off-gas of the reactor, and
wherein the PSA unit produces a hydrogen product stream comprising at least 98% by volume of hydrogen for feeding to the reactor and a PSA tail gas, wherein the PSA tail gas comprises between 5 and 60% by weight of C1-C3 alkanes which PSA tail gas is fed to the SMR to fuel the steam reforming process in the SMR unit.

7. Process according to claim 6, wherein the process further comprises carbon dioxide capture to capture at least part of the carbon dioxide produced by the hydrogen production section, the carbon dioxide capture comprising adsorption of carbon dioxide with methyldiethanol amine and regeneration of said methyldiethanol amine using steam.

8. Process according to claim 6 or 7, wherein the SMR produces steam at a pressure of between 5 and 70 bara, preferably between 10 and 50 bara.

9. Process according to any of claims 6 to 8, wherein the liquid product flow out of the reactor is subjected to one or more purification steps comprising one or more distillation steps and/or evaporation steps.

10. Process according to claim 8 or 9, wherein steam produced by the SMR is used for heating the reactor and/or for providing heat to one or more units of the purification steps.

11. Process according to any of claims 6 to 10, wherein one or more bottoms streams of distillation comprise polyols, and wherein one or more of these bottom streams is subjected to burning in a boiler to produce steam at a pressure of between 5 and 70 bara, preferably between 10 and 50 bara.

12. Process according to any of claims 6 to 11, wherein the tungsten-containing compound of the catalyst system comprises tungstic acid (H₂WO₄), a tungstate salt, tungsten oxide or mixtures thereof, wherein the tungstate salt is preferably an alkali metal tungstate.

13. Process according to any of claims 6 to 12, wherein said tungsten-containing compound of the catalyst system is continuously or periodically added to the reactor.

14. Process according to any of claims 6 to 13, wherein the hydrogenolysis metal selected from the groups 8, 9 or 10 of the Periodic Table of Elements is selected from the group consisting of Cu, Fe, Ni, Co, Pd, Pt, Ru, Rh, Ir, Os, and combinations thereof, and which is preferably selected from Ru and Rh.

15. Process according to any of claims 6 to 14, wherein the amount of off-gas from the reactor has a specific weight in ton / ton of monoethylene glycol produced in the process of between 0.05 and 0.70 ton / ton MEG.

## Patentansprüche

1. Verarbeitungsanlage zum Produzieren von Ethylenglykol und/oder Propylenglykol und/oder Glycerin in einer kontinuierlichen Weise aus einer Zufuhr, umfassend eine Kohlenhydratquelle in einer wässrigen Flüssigkeit und Wasserstoff, die Verarbeitungsanlage umfassend:
einen Reaktor, der einen flüssigen Produktstrom aus dem Reaktor und ein Abgas produziert,
einen nachgeschalteten Reinigungsabschnitt, umfassend eine oder mehrere Destillations- und/oder Verdampfungseinheiten, um den flüssigen Produktstrom aus dem Reaktor einem oder mehreren Destillations- und/oder Verdampfungsschritten zu unterziehen,
einen Wasserstoffproduktionsabschnitt, umfassend:
o eine Dampf-Methan-Reformer-Einheit (SMR-Einheit),
o eine Wasser-Gas-Shift-Reaktor-Einheit (WGS-Einheit),
o eine Druckwechseladsorptionseinheit (PSA-Einheit),
wobei die Zufuhr zu der PSA-Einheit den wasserstoffhaltigen Produktstrom der WGS-Einheit und das Abgas des Reaktors umfasst, und
wobei die PSA-Einheit einen Wasserstoffproduktstrom produziert, umfassend mindestens zu 95 Gew.-% Wasserstoff für das Zuführen in den Reaktor und ein PSA-Restgas, wobei das PSA-Restgas zwischen 5 und 60 Gew.-% C1-C3-Alkane umfasst, wobei das PSA-Restgas dem SMR zugeführt wird, um den Dampfreformierungsprozess in der SMR-Einheit anzutreiben.

2. Verarbeitungsanlage nach Anspruch 1, wobei die Anlage ferner eine Kohlendioxid-Auffangeinheit umfasst, umfassend eine Absorptionssäule und eine Regenerationssäule, und wobei die Einheit Methyldiethanolamin umfasst.

3. Verarbeitungsanlage nach Anspruch 1 oder 2, wobei der SMR neben Synthesegas Dampf bei einem Druck zwischen 5 und 70 Bara, vorzugsweise zwischen 10 und 50 Bara, produziert.

4. Verarbeitungsanlage nach einem der vorstehenden Ansprüche, wobei die Anlage ferner einen Kessel zum Verbrennen der Bodenströme eines oder mehrerer Destillationsschritte umfasst, wobei der Kessel dabei Dampf bei einem Druck zwischen 5 und 70 Bara, vorzugsweise zwischen 10 und 50 Bara, produziert.

5. Verarbeitungsanlage nach einem der Ansprüche 3 oder 4, wobei der produzierte Dampf zum Heizen des Reaktors und/oder zum Bereitstellen von Wärme für eine oder mehrere Einheiten des nachgeschalteten Reinigungsabschnitts verwendet wird.

6. Verfahren zum Produzieren von Ethylenglykol und/oder Propylenglykol und/oder Glycerin in einer kontinuierlichen Weise aus einer Zufuhr zu einem Reaktor, die Zufuhr umfassend eine Kohlenhydratquelle in einer wässrigen Flüssigkeit, wobei dem Reaktor ferner Wasserstoffgas zugeführt wird, der Reaktor umfassend ein Katalysatorsystem, das Katalysatorsystem umfassend eine wolframhaltige Verbindung und ein Hydrogenolysemetall, das aus den Gruppen 8, 9 oder 10 des Periodensystems der Elemente ausgewählt ist, wobei ein flüssiger Produktstrom aus dem Reaktor Glykole und ein oder mehrere Polyole umfasst, und der Reaktor ferner ein Abgas bereitstellt, umfassend Wasserstoff und Methan,
wobei das dem Reaktor zugeführte Wasserstoffgas durch einen Wasserstoffproduktionsabschnitt bereitgestellt wird, umfassend:
o eine Dampf-Methan-Reformer-Einheit (SMR-Einheit),
o eine Wasser-Gas-Shift-Reaktor-Einheit (WGS-Einheit),
o eine Druckwechseladsorptionseinheit (PSA-Einheit),
wobei die Zufuhr zu der PSA-Einheit den wasserstoffhaltigen Produktstrom der WGS-Einheit und das Abgas des Reaktors umfasst, und
wobei die PSA-Einheit einen Wasserstoffproduktstrom produziert, umfassend mindestens zu 98 Vol.-% Wasserstoff für das Zuführen in den Reaktor und ein PSA-Restgas, wobei das PSA-Restgas zwischen 5 und 60 Gew.-% C1-C3-Alkane umfasst, wobei das PSA-Restgas dem SMR zugeführt wird, um den Dampfreformierungsprozess in der SMR-Einheit anzutreiben.

7. Verfahren nach Anspruch 6, wobei das Verfahren ferner eine Kohlendioxidabscheidung umfasst, um mindestens einen Teil des Kohlendioxids abzuscheiden, das durch den Wasserstoffproduktionsabschnitt produziert wird, die Kohlendioxidabscheidung umfassend eine Adsorption von Kohlendioxid mit Methyldiethanolamin und eine Regeneration des Methyldiethanolamins unter Verwendung von Dampf.

8. Verfahren nach Anspruch 6 oder 7, wobei der SMR Dampf bei einem Druck zwischen 5 und 70 Bara, vorzugsweise zwischen 10 und 50 Bara, produziert.

9. Verfahren nach einem der Ansprüche 6 bis 8, wobei der flüssige Produktstrom aus dem Reaktor einem oder mehreren Reinigungsschritten unterzogen wird, umfassend einen oder mehrere Destillationsschritte und/oder Verdampfungsschritte.

10. Verfahren nach Anspruch 8 oder 9, wobei Dampf, der durch den SMR produziert wird, zum Heizen des Reaktors und/oder zum Bereitstellen von Wärme für eine oder mehrere Einheiten der Reinigungsschritte verwendet wird.

11. Verfahren nach einem der Ansprüche 6 bis 10, wobei ein oder mehrere Bodenströme der Destillation Polyole umfassen und wobei einer oder mehrere dieser Bodenströme einer Verbrennung in einem Kessel unterzogen werden, um Dampf bei einem Druck zwischen 5 und 70 Bara, vorzugsweise zwischen 10 und 50 Bara, zu produzieren.

12. Verfahren nach einem der Ansprüche 6 bis 11, wobei die wolframhaltige Verbindung des Katalysatorsystems Wolframsäure (H₂WO₄), ein Wolframsalz, Wolframoxid oder Mischungen davon umfasst, wobei das Wolframsalz vorzugsweise ein Alkalimetallwolframat ist.

13. Verfahren nach einem der Ansprüche 6 bis 12, wobei die wolframhaltige Verbindung des Katalysatorsystems kontinuierlich oder periodisch dem Reaktor zugegeben wird.

14. Verfahren nach einem der Ansprüche 6 bis 13, wobei das Hydrogenolysemetall, das aus den Gruppen 8, 9 oder 10 des Periodensystems der Elemente ausgewählt ist, aus der Gruppe ausgewählt ist, bestehend aus Cu, Fe, Ni, Co, Pd, Pt, Ru, Rh, Ir, Os und Kombinationen davon, und das vorzugsweise aus Ru und Rh ausgewählt ist.

15. Verfahren nach einem der Ansprüche 6 bis 14, wobei die Abgasmenge aus dem Reaktor ein spezifisches Gewicht in Tonne/Tonne von Monoethylenglykol, das in dem Verfahren produziert wird, zwischen 0,05 und 0,70 Tonne/Tonne MEG aufweist.

## Revendications

1. Équipement de traitement permettant de produire de l'éthylène glycol et/ou du propylène glycol et/ou du glycérol d'une manière continue à partir d'une charge d'alimentation comprenant une source de glucides dans un liquide aqueux et de l'hydrogène, l'équipement de traitement comprenant :
- un réacteur produisant un écoulement de produit liquide sortant du réacteur et un effluent gazeux,
- une section de purification en aval comprenant une ou plusieurs unités de distillation et/ou d'évaporation permettant de soumettre l'écoulement de produit liquide sortant du réacteur à une ou plusieurs étapes de distillation et/ou d'évaporation,
- une section de production d'hydrogène comprenant :
o une unité de vaporeformeur de méthane (SMR),
o une unité de réacteur de déplacement de gaz à l'eau (WGS),
o une unité d'adsorption modulée en pression (PSA),
dans lequel la charge d'alimentation vers l'unité PSA comprend le courant de produit comprenant de l'hydrogène de l'unité WGS et l'effluent gazeux du réacteur, et
dans lequel l'unité PSA produit un courant de produit d'hydrogène comprenant au moins 95 % en poids d'hydrogène pour alimentation au réacteur et un gaz résiduaire de PSA, dans lequel le gaz résiduaire de PSA comprend entre 5 et 60 % en poids d'alcanes en C1 à C3, ce gaz résiduaire de PSA étant alimenté au SMR pour alimenter en combustible le processus de vaporeformage dans l'unité SMR.

2. Équipement de traitement selon la revendication 1, dans lequel l'équipement comprend en outre une unité de capture de dioxyde de carbone comprenant une colonne d'absorption et une colonne de régénération et cette unité comprend de la méthyldiéthanol-amine.

3. Équipement de traitement selon la revendication 1 ou la revendication 2, dans lequel le SMR produit, à côté de gaz de synthèse, une vapeur à une pression comprise entre 5 et 70 bar (absolus), de préférence entre 10 et 50 bar (absolus).

4. Équipement de traitement selon l'une quelconque des revendications précédentes, dans lequel l'équipement comprend en outre une chaudière permettant de brûler les courants de fond d'une ou plusieurs étapes de distillation, ce faisant cette chaudière produit de la vapeur, à une pression comprise entre 5 et 70 bar (absolus), de préférence entre 10 et 50 bar (absolus).

5. Équipement de traitement selon de quelconques revendications 3 ou 4, dans lequel la vapeur produite est utilisée pour chauffer le réacteur et/ou pour fournir de la chaleur à une ou plusieurs unités de la section de purification en aval.

6. Procédé permettant de produire de l'éthylène glycol et/ou du propylène glycol et/ou du glycérol d'une manière continue à partir d'une charge d'alimentation vers un réacteur, la charge d'alimentation comprenant une source de glucides dans un liquide aqueux, le réacteur étant en outre alimenté avec de l'hydrogène gazeux, le réacteur comprenant un système de catalyseur, ledit système de catalyseur comprenant un composé contenant du tungstène et un métal d'hydrogénolyse choisi dans les groupes 8, 9 ou 10 du tableau périodique des éléments, dans lequel un écoulement de produit liquide sortant du réacteur comprend des glycols et un ou plusieurs polyols, et le réacteur fournit en outre un effluent gazeux comprenant de l'hydrogène et du méthane,
dans lequel l'hydrogène gazeux étant alimenté au réacteur est fourni par une section de production d'hydrogène comprenant :
o une unité de vaporeformeur de méthane (SMR),
o une unité de réacteur de déplacement de gaz à l'eau (WGS),
o une unité d'adsorption modulée en pression (PSA),
dans lequel la charge d'alimentation vers l'unité PSA comprend le courant de produit comprenant de l'hydrogène de l'unité WGS et l'effluent gazeux du réacteur, et
dans lequel l'unité PSA produit un courant de produit d'hydrogène comprenant au moins 98 % en volume d'hydrogène pour alimentation au réacteur et un gaz résiduaire de PSA, dans lequel le gaz résiduaire de PSA comprend entre 5 et 60 % en poids d'alcanes en C1 à C3, ce gaz résiduaire de PSA étant alimenté au SMR pour alimenter en combustible le processus de vaporeformage dans l'unité SMR.

7. Procédé selon la revendication 6, dans lequel le procédé comprend en outre une capture de dioxyde de carbone pour capturer au moins une partie du dioxyde de carbone produit par la section de production d'hydrogène, la capture de dioxyde de carbone comprenant une adsorption de dioxyde de carbone avec de la méthyldiéthanol-amine et une régénération de ladite méthyldiéthanol-amine à l'aide de vapeur.

8. Procédé selon la revendication 6 ou 7, dans lequel le SMR produit de la vapeur à une pression comprise entre 5 et 70 bar (absolus), de préférence entre 10 et 50 bar (absolus).

9. Procédé selon l'une quelconque des revendications 6 à 8, dans lequel l'écoulement de produit liquide sortant du réacteur est soumis à une ou plusieurs étapes de purification comprenant une ou plusieurs étapes de distillation et/ou étapes d'évaporation.

10. Procédé selon la revendication 8 ou 9, dans lequel la vapeur produite par le SMR est utilisée pour chauffer le réacteur et/ou pour fournir de la chaleur à une ou plusieurs unités des étapes de purification.

11. Procédé selon l'une quelconque des revendications 6 à 10, dans lequel un ou plusieurs courants de fond de distillation comprennent des polyols, et dans lequel un ou plusieurs de ces courants de fond sont soumis à une combustion dans une chaudière pour produire de la vapeur à une pression comprise entre 5 et 70 bar (absolus), de préférence entre 10 et 50 bar (absolus).

12. Procédé selon l'une quelconque des revendications 6 à 11, dans lequel le composé contenant du tungstène du système de catalyseur comprend de l'acide tungstique (H₂WO₄), un sel tungstate, de l'oxyde de tungstène ou des mélanges de ceux-ci, dans lequel le sel tungstate est de préférence un tungstate de métal alcalin.

13. Procédé selon l'une quelconque des revendications 6 à 12, dans lequel ledit composé contenant du tungstène du système de catalyseur est ajouté de façon continue ou périodique au réacteur.

14. Procédé selon l'une quelconque des revendications 6 à 13, dans lequel le métal d'hydrogénolyse choisi dans les groupes 8, 9 ou 10 du tableau périodique des éléments est choisi dans le groupe constitué par Cu, Fe, Ni, Co, Pd, Pt, Ru, Rh, Ir, Os, et des combinaisons de ceux-ci, et celui-ci est choisi de préférence parmi Ru et Rh.

15. Procédé selon l'une quelconque des revendications 6 à 14, dans lequel la quantité d'effluent gazeux provenant du réacteur a un poids spécifique en tonne/tonne de monoéthylène glycol produit dans le procédé compris entre 0,05 et 0,70 tonne/tonne de MEG.
